# EUROPÄISCHE PATENTANMELDUNG

(11) **EP 3 925 563 A1**
(43) Veröffentlichungstag der Anmeldung: **22.12.2021**
(21) Anmeldenummer: 21178931.8
(22) Anmeldetag: 11.06.2021
(51) Int. Cl.: A61B 34/37, A61B 46/10, A61B 90/98, A61B 90/00, A61B 17/29, A61B 1/00, A61B 34/00

(54) **VORRICHTUNG ZUR ROBOTERGESTÜTZTEN CHIRURGIE**

(30) Priorität: 19.06.2020 DE 102020116256
(71) Anmelder: avateramedical GmbH, 07745 Jena (DE)
(72) Erfinder: Keim, Tobias, 07343 Wurzbach (DE); Jahn, Michael, 07768 Kahla, OT Kleineutersdorf (DE); Baudisch, Tobias Django, 07745 Jena (DE)
(74) Vertreter: Schaumburg und Partner Patentanwälte mbB

(57) **Zusammenfassung**

Eine Vorrichtung zur robotergestützten Chirurgie umfasst mindestens einen Manipulatorarm (16a bis 16d) mit einer nicht sterilen Koppeleinheit (44a, 204, 304, 610), die mindestens ein erstes Antriebselement (62, 202, 302, 612, 614, 616, 618) umfasst. Weiterhin umfasst die Vorrichtung eine in einem sterilen Bereich (40) angeordnete sterile Instrumenteneinheit (100a bis 100d, 200, 300, 500, 600), die mindestens ein zweites Antriebselement (54, 60, 208, 308, 502, 504, 602, 604, 606, 608) umfasst, das um eine Rotationsachse (52) rotierbar angeordnet ist. Das erste Antriebselement (62, 202, 302, 612, 614, 616, 618) und das zweite Antriebselement (54, 60, 208, 308, 502, 504, 602, 604, 606, 608) sind derart ausgebildet und in einem gekoppelten Zustand derart angeordnet, dass durch das erste Antriebselement (62, 202, 302, 612, 614, 616, 618) eine Kraft auf das zweite Antriebselement (54, 60, 208, 308, 502, 504, 602, 604, 606, 608), zur Rotation des zweiten Antriebselements (54, 60, 208, 308, 502, 504, 602, 604, 606, 608) um die Rotationsachse (52), ausübbar ist und im gekoppelten Zustand das erste Antriebselement (62, 202, 302, 612, 614, 616, 618) und das zweite Antriebselement (54, 60, 208, 308, 502, 504, 602, 604, 606, 608) sind in einer Rotationsebene (55) senkrecht zur Rotationsachse (52) nebeneinander angeordnet. Weiterhin umfasst die Vorrichtung eine Sterilbarriere (42,64), die zumindest zwischen dem ersten Antriebselement (62, 202, 302, 612, 614, 616, 618) und dem zweiten Antriebselement (54, 60, 208, 308, 502, 504, 602, 604, 606, 608) angeordnet ist.

## Beschreibung

Die Erfindung betrifft eine Vorrichtung zur robotergestützten Chirurgie mit mindestens einem Manipulatorarm mit einer nicht sterilen Koppeleinheit, die mindestens ein erstes Antriebselement hat. Die Vorrichtung hat weiterhin mindestens eine in einem sterilen Bereich angeordnete sterile Instrumenteneinheit, die mindestens ein zweites Antriebselement umfasst, das um eine Rotationsachse rotierbar angeordnet ist. Die Instrumenteneinheit ist mit der Koppeleinheit koppelbar.

In der minimalinvasiven Chirurgie werden zunehmend sogenannte Telemanipulatorsysteme, die auch als Roboterassistenzsysteme bezeichnet werden, zur robotergestützten Chirurgie eingesetzt. Das sterile Operationsfeld wird vor den nicht sterilen Elementen des Telemanipulatorsystems mit Hilfe einer sterilen Abdeckung geschützt. Durch die sterile Abdeckung wird sowohl eine Kontamination des sterilen Operationsfeldes als auch eine Verschmutzung des Telemanipulatorsystems durch Körperflüssigkeiten und/oder Gewebe des operierten Patienten oder des Operationspersonals verhindert. Dadurch wird das Risiko von Kreuzkontaminationen verringert.

Mit Hilfe des Telemanipulatorsystems werden chirurgische Instrumente und/oder Endoskope aufgrund von Bedieneingaben in ihrer Lage und Orientierung gesteuert und kommen dabei zwangsläufig in Körperkontakt mit dem zu operierenden Patienten, sodass die chirurgischen Instrumente und/oder Endoskope mit Körperflüssigkeiten und/oder Gewebe des operierten Patienten verunreinigt werden. Gleichzeitig müssen die chirurgischen Instrumente mechanisch, elektrisch und/oder optisch an das Telemanipulatorsystem angekoppelt werden, um eine aktive Positionierung und Ausrichtung des chirurgischen Instruments sowie eine gewünschte Betätigung eines chirurgischen Instruments realisieren zu können. Hierzu ist an jedem Manipulatorarm eine Koppelschnittstelle, die als Koppeleinheit ausgebildet sein kann, vorgesehen.

Das während eines chirurgischen Eingriffs genutzte Material einschließlich der verwendeten chirurgischen Apparate und Instrumente und die weiteren Bestandteile des Telemanipulatorsystems können in drei Kategorien eingeteilt werden:
Kategorie 1: Das Material ist steril und wird während des chirurgischen Eingriffs kontaminiert. Das Material wird nach der Operation entsorgt. Es erfolgt somit eine Einmalverwendung des Materials.
Kategorie 2: Das Material ist steril, wird während des chirurgischen Eingriffs kontaminiert und nach der Operation gereinigt und sterilisiert. Es erfolgt somit eine Mehrfachverwendung des Materials. Solche mehrfach verwendeten Materialien müssen entsprechend den Anforderungen an eine prozessfähige Sterilisierbarkeit konstruiert und produziert sein.
Kategorie 3: Das Material ist nicht steril. Während des chirurgischen Eingriffs wird durch eine sterile Abdeckung und Umverpackung eine Kontamination des sterilen Operationsfeldes verhindert. Gleichzeitig wird das nicht sterile Material vor Kontakt mit Körperflüssigkeiten und/oder Gewebe geschützt.

Ist es erforderlich, Geräte der Kategorie 1 oder der Kategorie 2 mit Geräten der Kategorie 3 zu koppeln, ist eine Sterilschnittstelle und/oder eine Sterilbarriere erforderlich, die eine Kontamination der Geräte der Kategorie 1 bzw. der Kategorie 2 durch die nicht sterilen Geräte der Kategorie 3 verhindert und umgekehrt eine Verunreinigung der Geräte der Kategorie 3 verhindert, da diese im allgemeinen als nicht sterilisierbare und nicht autoklavierbare Komponenten technisch ausgeführt sind. Die Ausführung von Geräten als sterilisierbare und autoklavierbare Komponenten erfordert eine besondere technische Auslegung des Geräts für den Sterilisationsprozess, sodass hierfür ein höherer Entwicklungsaufwand sowie ein erheblicher Validierungsaufwand zum Nachweis der Wirksamkeit des Sterilisationsprozesses erforderlich sind. Für einen solchen Nachweis ist es insbesondere erforderlich, das Gerät mehrfach nacheinander zu kontaminieren, zu sterilisieren, eine Wirksamkeitsprüfung der Sterilisation durchzuführen sowie eine Funktionsprüfung nach erfolgter Sterilisation durchzuführen. Dabei ist ein Nachweis zu erbringen, dass die Geräte nach jeder Sterilisation sicher sterilisiert und somit wiederverwendet werden könnten.

Aus dem Dokument US 7,666,191 B1 ist ein Telemanipulatorsystem bekannt, bei dem die nicht sterilen Manipulatorarme mittels einer Sterilfolie abgedeckt werden. Die Koppeleinheit des Manipulatorarms umfasst vier Rotationsaktuatoren, die mit einer ersten Seite eines in der Sterilfolie integrierten Steriladapters gekoppelt werden. Mit Hilfe des Steriladapters werden die Drehbewegungen der vier Rotationsaktuatoren der Koppeleinheit des Manipulatorarms in Eingriff mit vier in den Steriladapter integrierten, drehbar gelagerten Übertragungsmittel gekoppelt. An der sterilen Außenseite des Steriladapters können diese sterilen Übertragungsmittel mit angetriebenen Elementen des sterilen chirurgischen Instruments in Eingriff gebracht werden. Ferner können über diesen Steriladapter elektrische Signale zwischen der Innenseite und der Außenseite des Steriladapters übertragen werden.

Somit wird mit Hilfe des Steriladapters verhindert, dass die Rotationsaktuatoren und die elektrischen Anschlüsse des sterilen chirurgischen Instruments direkt in Kontakt mit den Rotationsaktuatoren und den elektrischen Anschlüssen der Koppeleinheit des nicht sterilen Manipulatorarms kommen. Eine Kontamination des chirurgischen Instruments durch den Kontakt mit nicht sterilen Teilen des Manipulatorarms wird durch den Steriladapter verhindert. Bei dieser Lösung ist es jedoch erforderlich, dass der Steriladapter drehbar gelagerte Übertragungsmittel haben muss sowie Übertragungsmittel zur Übertragung von elektrischen Signalen, wodurch der Adapter aufwendig herzustellen und störanfällig ist. Insbesondere ist es aufwendig, die Drehbarkeit der Übertragungsmittel und die Undurchlässigkeit des Lagers der Übertragungsmittel in dem Steriladapter sicherzustellen, wenn die Übertragungsmittel in Kontakt mit Körperflüssigkeit kommen. Der Steriladapter selbst ist als Teil der Sterilfolie zur Einmalverwendung vorgesehen.

Aus dem Dokument EP 3025667 A1 ist eine Sterilschleuse bekannt, die Antriebselemente einer Koppeleinheit von einem sterilen Operationsbereich steril abschirmt bevor eine sterile Instrumenteneinheit mit der Sterilschleuse verbunden wird und nachdem die sterile Instrumenteneinheit von der Sterilschleuse getrennt wird. Weiterhin hat die sterile Instrumenteneinheit Sterilklappen, die Antriebselement der Instrumenteneinheit von einem sterilen Operationsbereich steril abschirmt bevor die sterile Instrumenteneinheit mit der Sterilschleuse verbunden wird und nachdem die sterile Instrumenteneinheit von der Sterilschleuse getrennt wird.

Grundsätzlich ist jedes Element in der Funktionskette zur Kopplung des Manipulatorarms und des Instruments eine mögliche Fehlerquelle und ist mit zusätzlichen Kosten verbunden. Je komplexer eine Sterilschleuse oder ein Steriladapter ausgebildet sind, insbesondere je mehr bewegliche Elemente vorgesehen sind, desto mehr Fehlerquellen ergeben sich. Weiterhin haben die bekannten Sterilschleusen und Steriladapter Lager und/oder Anstoßflächen von Klappen, deren Undurchlässigkeit sichergestellt sein muss.

Es ist Aufgabe der Erfindung eine Vorrichtung zur robotergestützten Chirurgie anzugeben, bei der eine sterile Kopplung einer Koppeleinheit eines unsterilen Manipulatorarms mit einer in einem sterilen Bereich angeordneten sterilen Instrumenteneinheit einfach möglich ist, wobei eine zuverlässige Kraftübertragung zwischen der Koppeleinheit und der Instrumenteneinheit möglich ist und eine Sterilbarriere unsterile Elemente von dem sterilen Bereich abschirmt.

Diese Aufgabe wird durch eine Vorrichtung mit den Merkmalen des Anspruchs 1 gelöst. Vorteilhafte Weiterbildungen sind in den abhängigen Ansprüchen angegeben.

Eine Vorrichtung zur robotergestützten Chirurgie umfasst mindestens einen Manipulatorarm mit einer nicht sterilen Koppeleinheit, die mindestens ein erstes Antriebselement umfasst. Weiterhin umfasst die Vorrichtung eine in einem sterilen Bereich angeordnete sterile Instrumenteneinheit, die mindestens ein zweites Antriebselement umfasst, das um eine Rotationsachse rotierbar angeordnet ist, wobei die Instrumenteneinheit mit der Koppeleinheit des Manipulatorarms koppelbar ist. Das erste Antriebselement und das zweite Antriebselement sind derart ausgebildet und in einem gekoppelten Zustand derart angeordnet, dass durch das erste Antriebselement eine Kraft auf das zweite Antriebselement, zur Rotation des zweiten Antriebselements um die Rotationsachse, ausübbar ist. Im gekoppelten Zustand sind das erste Antriebselement und das zweite Antriebselement in einer Rotationsebene senkrecht zur Rotationsachse nebeneinander angeordnet. Weiterhin umfasst die Vorrichtung eine Sterilbarriere, die zumindest zwischen dem ersten Antriebselement und dem zweiten Antriebselement angeordnet ist. Dadurch wird eine besonders kompakte und einfache Bauweise ermöglicht. Insbesondere ist die Bauweise der Sterilbarriere besonders einfach. Das erste Antriebselement kann auch als Koppeleinheitsantriebselement und das zweite Antriebselement als Instrumentenantriebselement bezeichnet werden.

Es ist vorteilhaft, wenn im gekoppelten Zustand das erste Antriebselement in der Rotationsebene des zweiten Antriebselements so angeordnet ist, dass eine dem zweiten Antriebselement zugewandte Oberfläche des ersten Antriebselements in zumindest einem Bereich parallel zu einer zylindrischen Mantelfläche oder einer zylindrischen Hüllmantelfläche des zweiten Antriebselements verläuft. Dadurch wird es ermöglicht, dass das erste Antriebselement die Kraft auf das zweite Antriebselement besonders sicher ausüben kann

Es ist vorteilhaft, wenn in der Rotationsebene an einer dem zweiten Antriebselement zugewandten Seite des ersten Antriebselements magnetfelderzeugende Elemente angeordnet sind und auf einer Kreisbahn um die Rotationsachse magnetfelderzeugende oder magnetische Elemente des zweiten Antriebselements angeordnet sind. Dadurch wird ein besonders sicherer Antrieb des zweiten Antriebselements ermöglicht.

Es ist besonders vorteilhaft, wenn die magnetfelderzeugenden Elemente des ersten Antriebselements Elektromagnete sind und die magnetfelderzeugenden Elemente des zweiten Antriebselements Elektromagnete mit umlaufend abwechselnden Polen sind, oder wenn die magnetfelderzeugenden Elemente des ersten Antriebselements Elektromagnete sind und die magnetischen Elemente des zweiten Antriebselements Permanentmagnete mit umlaufend abwechselnden Polen sind. Dadurch wird eine besonders kompakte und robuste Bauweise ermöglicht.

Es ist besonders vorteilhaft, wenn in dem ersten Antriebselement mit Hilfe der Elektromagnete Magnetfelder erzeugbar sind, durch die die Kraft auf die Permanentmagnete des zweiten Antriebselements ausübbar ist, wobei durch Änderung der Polung der Elektromagnete und/oder Änderung eines Schaltzustands der Elektromagnete das zweite Antriebselement rotiert. Dadurch wird ein zuverlässiger Antrieb des zweiten Antriebselements ermöglicht.

Es ist vorteilhaft, wenn das erste Antriebselement mindestens einen beweglichen und/oder verformbaren Aktor umfasst, der so bewegbar und/oder verformbar ist, dass der Aktor eine Mantelfläche des zweiten Antriebselements zumindest zeitweise derart kontaktiert, dass durch Kraftschluss und/oder Formschluss zwischen dem Aktor und der Mantelfläche des zweiten Antriebselements, die Kraft auf das zweite Antriebselement ausübbar ist. Dadurch wird ein eine besonders sichere Kraftübertragung vom ersten auf das zweite Antriebselement erreicht.

Es ist besonders vorteilhaft, wenn der Aktor mindestens ein piezoelektrischer Aktor ist und mit dem, durch Kraftschluss und/oder Formschluss zwischen dem Aktor und der Mantelfläche des zweiten Antriebselements, die Kraft auf das zweite Antriebselement ausübbar ist. Dadurch wird ein besonders leistungsstarker Antrieb des zweiten Antriebselements erreicht.

Es ist vorteilhaft, wenn die dem zweiten Antriebselement zugewandte Oberfläche des ersten Antriebselements entlang eines Kreisbogens um die Rotationsachse mit einem Mittelpunktswinkel von 45° bis 180° verläuft. Dadurch wird ein besonders robuster Antrieb des zweiten Antriebselements sichergestellt.

Es ist vorteilhaft, wenn die Sterilbarriere zumindest im Bereich zwischen dem ersten Antriebselement und dem zweiten Antriebselement eine durchgehend geschlossene Oberfläche hat. Dadurch wird eine besonders hohe Sicherheit zur Verhinderung einer Kontamination des sterilen Bereichs sichergestellt.

Es ist vorteilhaft, wenn ein Abschnitt der zwischen dem ersten Antriebselement und dem zweiten Antriebselement angeordneten Sterilbarriere entlang eines Kreisbogens um die Rotationsachse herum verläuft. Dadurch wird eine besonders sichere Anordnung der Sterilbarriere erreicht.

Es ist vorteilhaft, wenn die Koppeleinheit mindestens eine Sensoreinheit zum Detektieren eines Drehwinkels des zweiten Antriebselements umfasst. Dadurch wird eine besonders genaue Kontrolle über die Bewegung des zweiten Antriebselement erreicht.

Es ist vorteilhaft, wenn die Sensoreinheit einen optischen Sensor umfasst, der ein in einer Rotationsebene umlaufendes, codiertes, optisch erfassbares Muster auf dem zweiten Antriebselement erfasst, wobei jedem erfassbaren Drehwinkel des zweiten Antriebselements ein bei diesem Drehwinkel durch den Sensor erfassbarer Teil des Musters zugeordnet ist, wobei sich jeder durch den Sensor bei einem Drehwinkel erfassbare Teil des Musters eindeutig von anderen durch den Sensor bei anderen Drehwinkeln erfassbaren Teilen des Musters unterscheidet. Dadurch wird eine besonders sichere Erfassung des Drehwinkels erreicht.

Es ist vorteilhaft, wenn in einem gekoppelten Zustand die zweiten Antriebselemente in mehreren Rotationsebenen übereinander entlang derselben Rotationsachse angeordnet sind und jedes erste Antriebselement in einer Rotationsebene mit einem zweiten Antriebselement paarweise angeordnet ist, wobei die Anzahl der Paare von Antriebselementen mindestens zwei ist, insbesondere drei oder vier ist. Dadurch sind eine Vielzahl von Freiheitsgraden eines Endeffektors der Instrumenteneinheit möglich.

Es ist besonders vorteilhaft, wenn mehrere Sensoreinheiten vorgesehen sind, dass die Anzahl der Sensoreinheiten mindestens der Anzahl von zweiten Antriebselementen entspricht, und dass jeweils mindestens eine Sensoreinheit den Drehwinkel eines zweiten Antriebselements erfasst. Dadurch wird eine besonders genaue und sichere Steuerung des Endeffektors erreicht.

Es ist vorteilhaft, wenn die Instrumenteneinheit ein Instrument mit einem an einem distalen Ende eines Instrumentenschafts angeordneten Endeffektor umfasst, wobei das mindestens eine zweite Antriebselement mit dem Endeffektor gekoppelt ist und wobei der Endeffektor mit Hilfe des mindestens einen zweiten Antriebselements in mindestens einem Freiheitsgrad bewegbar und/oder steuerbar ist, insbesondere bei vier zweiten Antriebselementen in vier Freiheitsgraden bewegbar ist, wobei jeweils zwei der zweiten Antriebselemente eine Drehbewegung um die Längsachse des Instrumentenschafts bewirken und jeweils zwei weitere zweite Antriebselemente eine Längsbewegung in Richtung der Längsachse des Instrumentenschafts bewirken. Alternativ ist es vorteilhaft, wenn die Instrumenteneinheit ein Endoskop mit einem Endoskopschaft umfasst, wobei das mindestens eine zweite Antriebselement mit dem Endoskop, dem Endoskopschaft und/oder einer Optik des Endoskops derart gekoppelt ist, dass mit Hilfe des mindestens einen zweiten Antriebselements in mindestens einem Freiheitsgrad eine Bewegung des Endoskops, des Endoskopschafts und/oder der Optik möglich ist. Dadurch wird eine besonders große Vielfalt an verschiedenen Instrumenteneinheiten ermöglicht und eine besonders flexible Verwendung der Vorrichtung ermöglicht.

Weitere Merkmale und Vorteile ergeben sich aus der folgenden Beschreibung, welche in Verbindung mit den beigefügten Figuren Ausführungsbeispiele näher erläutert.

Es zeigen:
- Fig. 1: eine schematische Darstellung einer Vorrichtung zur robotergestützten Chirurgie,
- Fig. 2: einen Teil eines Manipulatorarms der Vorrichtung nach Figur 1 mit einer Koppeleinheit sowie einer Sterilbarriere und einer Instrumenteneinheit gemäß einer ersten Ausführungsform in einem nicht gekoppelten Zustand,
- Fig. 3: einen Teil des Manipulatorarms mit der Koppeleinheit sowie der Sterilbarriere und der Instrumenteneinheit nach Figur 2 in einem gekoppelten Zustand,
- Fig. 4: eine schematische Explosionszeichnung der Instrumenteneinheit,
- Fig. 5: eine Seitenansicht der Instrumenteneinheit,
- Fig. 6: eine Instrumenteneinheit gemäß einer zweiten Ausführungsform,
- Fig. 7: eine Instrumenteneinheit gemäß einer dritten Ausführungsform,
- Fig. 8: eine Koppeleinheit und eine Instrumenteneinheit mit einem elektromagnetischen Antrieb gemäß einer vierten Ausführungsform,
- Fig. 9: eine schematische Zeichnung von magnetfelderzeugenden Elementen eines elektromagnetischen Antriebs der Koppeleinheit und der Instrumenteneinheit nach Figur 8 in einem ersten Zustand mit einer ersten Polarisierung,
- Fig. 10: eine schematische Zeichnung des elektromagnetischen Antriebs nach Figur 9 in einem zweiten Zustand mit einer zweiten Polarisierung,
- Fig. 11: eine Koppeleinheit und eine Instrumenteneinheit gemäß einer fünften Ausführungsform mit einer mechanischen Kraftübertragung,
- Fig. 12: eine schematische Zeichnung von Eingriffselementen der Koppeleinheit und der Instrumenteneinheit nach Figur 11 in einem ersten Schritt,
- Fig. 13: eine schematische Zeichnung der Eingriffselemente nach Figur 12 in einem zweiten Schritt,
- Fig. 14: eine Koppeleinheit mit vier Koppeleinheitsantriebselementen und der Instrumenteneinheit gemäß der dritten Ausführungsform,
- Fig. 15: eine schematische Schnittzeichnung eines Instrumentenantriebselements gemäß einer sechsten Ausführungsform einer Instrumenteneinheit, und
- Fig. 16: eine schematische Darstellung eines Antriebs einer Instrumenteneinheit.

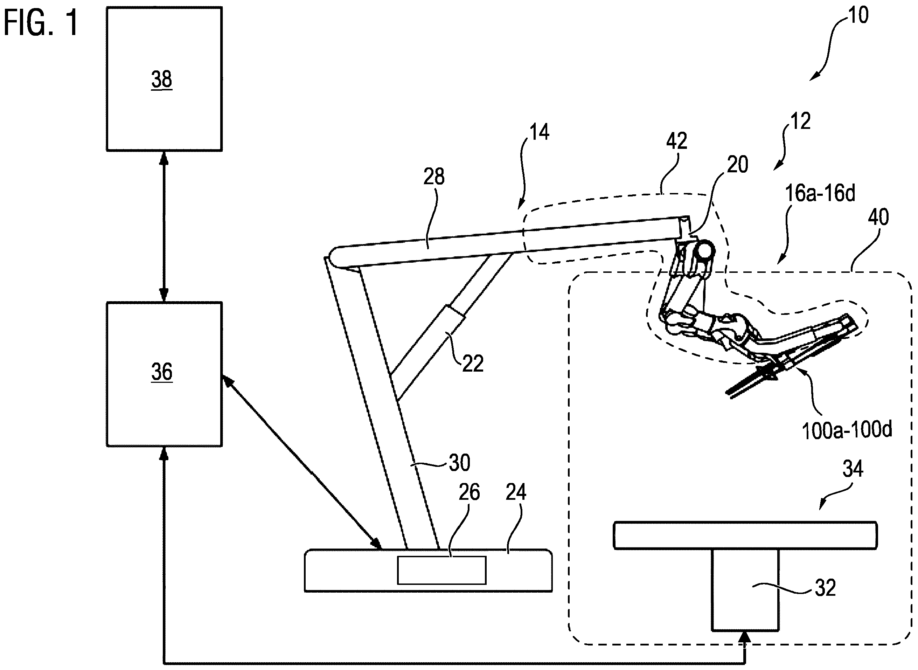

Figur 1 zeigt eine schematische Darstellung einer Vorrichtung 10 zur robotergestützten Chirurgie mit einem Manipulator 12, der ein Stativ 14 und vier Manipulatorarme 16a bis 16d hat. Bei anderen Ausführungsbeispielen kann der Manipulator 12 auch mehr oder weniger Manipulatorarme 16a bis 16d haben. Jeder Manipulatorarm 16a bis 16d ist mit einer Instrumenteneinheit 100a bis 100d über eine Koppeleinheit des Manipulatorarms 16a bis 16d verbunden. Die Instrumenteneinheit 100a bis 100d ist steril und umfasst ein chirurgisches Instrument, insbesondere mit einem Endeffektor, wobei der Endeffektor mit Hilfe der Koppeleinheit des Manipulatorarms 16a bis 16d bewegt und/oder betätigt werden kann. Alternativ zum chirurgischen Instrument kann die Instrumenteneinheit 100a bis 100d auch ein optisches Instrument, insbesondere ein Endoskop, und/oder ein medizinisches Gerät, insbesondere zum Applizieren eines Medikaments, zum Abgeben einer Spülflüssigkeit und/oder zum Absaugen von Spülflüssigkeit und/der Sekret, umfassen.

Das Stativ 14 hat ein auf dem Fußboden eines Operationssaals stehenden Stativfuß 24. Die Manipulatorarme 16a bis 16d sind mit einem Stativkopf 20 des Stativs 14 verbunden. Bei anderen Ausführungsformen kann das Stativ auch ein Deckenstativ sein.

Die Position des Stativkopfs 20 ist mit Hilfe einer Stativarmantriebseinheit 22 und mit einer in dem Stativfuß 24 angeordneten Stativfußantriebseinheit 26 einstellbar. Mit Hilfe der Antriebseinheit 22 sind Stativarme 28, 30 relativ zueinander bewegbar. Mit Hilfe der Antriebseinheit 26 kann die Neigung des Stativarms 30 relativ zur Aufstellfläche des Stativfußes 24 geändert und/oder der Stativarm 30 um eine senkrechte Drehachse gedreht werden. Im Allgemeinen erfolgt die Positionierung des Stativkopfs 20 vor einer Operation eines Patienten. Während der Operation bleibt die Lage des Stativkopfs 20 zu der Säule 32 eines Operationstischs 34 üblicherweise unverändert. Der Manipulator 12 wird mit Hilfe einer Steuereinheit 36 gesteuert. Die Steuereinheit 36 ist über eine Daten- und/oder Steuerleitung mit einer Ein- und Ausgabeeinheit 38 verbunden, die insbesondere ein Bild des Operationsfelds an eine Bedienperson in Echtzeit mit Hilfe mindestens einer Anzeigeeinheit ausgibt. Die Bedienperson macht Bedieneingaben, durch die die Instrumenteneinheiten 100a bis 100d während der Operation des Patienten positioniert und betätigt werden. Die Ein- und Ausgabeeinheit 38 dient somit als Mensch-Maschinen-Schnittstelle.

Die Steuereinheit 36 ist ferner über eine Steuer- und/oder Datenverbindung mit einer nicht dargestellten Steuereinheit des Operationstischs 34 verbunden. Über diese Steuer- und/oder Datenverbindung wird unter anderem sichergestellt, dass die Lage der Patientenlagerfläche oder von Segmenten der Patientenlagerfläche des Operationstischs 34 nur dann verändert werden kann, wenn dies aufgrund der Positionierung der Instrumenteneinheiten 100a bis 100d für einen zu operierenden Patienten gefahrlos möglich ist.

Der Operationstisch 34 sowie die Instrumenteneinheiten 100a bis 100d sind in einem sterilen Operationsbereich 40 angeordnet. Die Manipulatorarme 16a bis 16d und das Stativ 14 sind nicht steril. Die in dem sterilen Operationsbereich 40 ragenden Bereiche des Manipulators 12, d.h. die Manipulatorarme 16a bis 16d mit Koppeleinheiten, der Stativkopf 20 und ein Teil des Stativarms 28, sind in einer mit Hilfe der Strichlinie angedeuteten Sterilbarriere 42, wie beispielsweise einer sterilen flexiblen Hülle oder einer Sterilfolie, steril verpackt, sodass sie gefahrlos im sterilen Operationsbereich 40 angeordnet werden können. Die Ein- und Ausgabeeinheit 38 ist außerhalb des sterilen Bereichs 40 angeordnet und muss deshalb nicht steril verpackt werden.

Bei einer Vielzahl von Operationen müssen die Instrumenteneinheiten 100a bis 100d während der Operation aufgrund des Operationsverlaufs mehrfach gewechselt werden. Somit muss zwischen dem Manipulatorarm 16a bis 16d und der Instrumenteneinheit 100a bis 100d eine sterile Schnittstelle vorgesehen werden, die sicherstellt, dass die nicht sterile Koppeleinheit des Manipulatorarms 16a bis 16d auch nach dem Trennen der Instrumenteneinheit 100a bis 100d steril abgedeckt sind.

Darüber hinaus darf die sterile Instrumenteneinheit 100a bis 100d nicht mit unsterilen Teilen der Koppeleinheit oder des Manipulatorarms 16a bis 16d in direkten Kontakt kommen um eine Kontaminierung der sterilen Instrumenteneinheit 100a bis 100d und des sterilen Bereichs 40 vor und/oder nach dem Trennen der Instrumenteneinheit 100a bis 100d vom Manipulatorarm 16a bis 16d zu verhindern. Damit kann die Instrumenteneinheit 100a bis 100d im sterilen Bereich 40 abgelegt werden, ohne weitere Elemente im sterilen Bereich 40 zu kontaminieren. Die Sterilbarriere 42 ist so ausgebildet, dass sie den Manipulatorarm 16a bis 16d, die Koppeleinheit, den Stativkopf 20 und zumindest Teile des Stativarms 28 verpackt und vom sterilen Bereich hermetisch trennt und abschirmt. Dafür kann die Sterilbarriere 42 aus mehreren einzelnen Elementen zusammengefügt sein, wobei Nähte zwischen den einzelnen Elementen so gefertigt sind, beispielsweise geschweißt oder geklebt sind, dass sie undurchlässig sind und eine sterile Trennung des sterilen Bereichs von unsterilen Einheiten erlauben. Alternativ ist die Sterilbarriere 42 nahtlos aus einem Stück gefertigt.

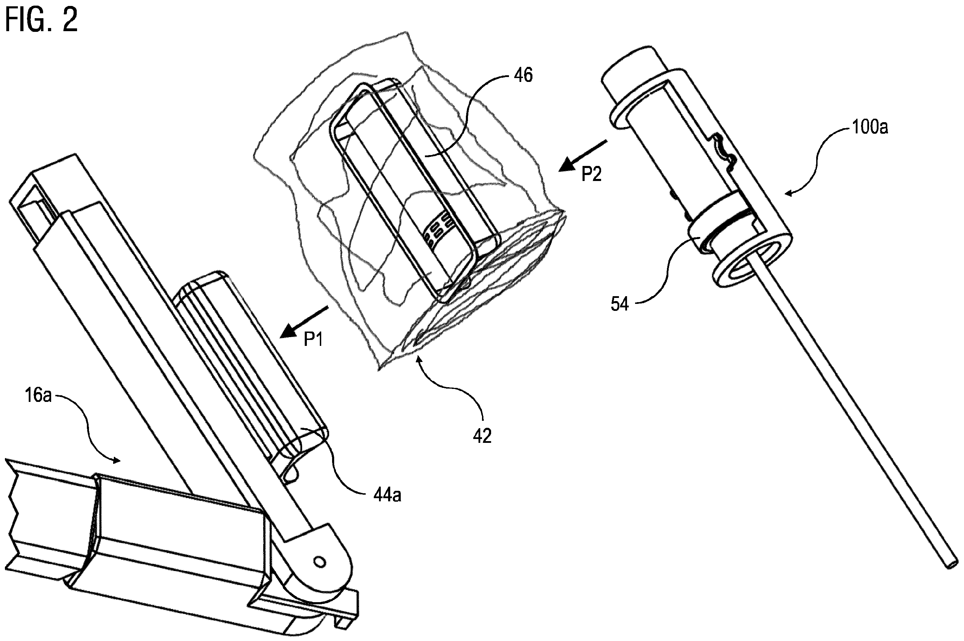

Figur 2 zeigt einen Teil des Manipulatorarms 16a der Vorrichtung nach Figur 1 mit einer Koppeleinheit 44a sowie die Sterilbarriere 42 und die Instrumenteneinheit 100a in einem nicht gekoppelten Zustand. Elemente mit gleichem Aufbau und/oder gleicher Funktion haben in weiteren Figuren dieselben Bezugszeichen. Im Folgenden wird nur der Manipulatorarm 16a mit der Instrumenteneinheit 100a und der Koppeleinheit 44a beschrieben. Die Ausführungen gelten jedoch auch für die im Wesentlichen gleich aufgebauten Manipulatorarme 16b bis 16d sowie die mit ihnen verbundenen Instrumenteneinheiten 100b bis 100d.

Die Koppeleinheit 44a umfasst ein in Figur 2 nicht sichtbares Koppeleinheitsantriebselement. Die Instrumenteneinheit 100a umfasst ein entsprechendes Instrumentenantriebselement 54. Das Koppeleinheitsantriebselement kann allgemein auch als erstes Antriebselement und das Instrumentenantriebselement 54 allgemein auch als zweites Antriebselement bezeichnet werden.

Die Sterilbarriere 42 befindet sich in Figur 2 in einem zusammengefalteten Zustand. Im Ausführungsbeispiel nach Figur 2 umfasst die Sterilbarriere 42 einen flexiblen Teil, beispielsweise eine Sterilfolie, sowie einen formstabilen Teil 46. Der formstabile Teil 46 der Sterilbarriere 42 ist dabei passgenau gefertigt und zumindest bereichsweise soweit elastisch verformbar, dass er über die Koppeleinheit 44a gezogen werden kann und in einem zusammengefügten Zustand eng an zumindest einem Teil einer Hüllfläche der Koppeleinheit 44a anliegt. Insbesondere liegt eine Seite des Teils 46 eng an der der Instrumenteneinheit 100a zugewandten Oberfläche der Koppeleinheit 44a an. Der flexible Teil der Sterilbarriere 42 wird über den Manipulatorarm 16a gezogen.

Der flexible Teil der Sterilbarriere 42 und der formstabile Teil 46 sind miteinander verbunden, sodass sie eine durchgehende sterile Barriere zwischen dem sterilen Bereich 40 und dem Manipulatorarm 16a, der Koppeleinheit 44a und dem Stativarm 28 bilden.

Vor dem Koppeln der Instrumenteneinheit 100a mit der Koppeleinheit 44a wird die Sterilbarriere 42 über den Manipulatorarm 16a gezogen und der formstabile Teil 46 in Richtung des Pfeils P1 über die Koppeleinheit 44a gezogen. Zum Koppeln wird anschließend die Instrumenteneinheit 100a in Richtung des Pfeils P2 mit der Koppeleinheit 44a verbunden.

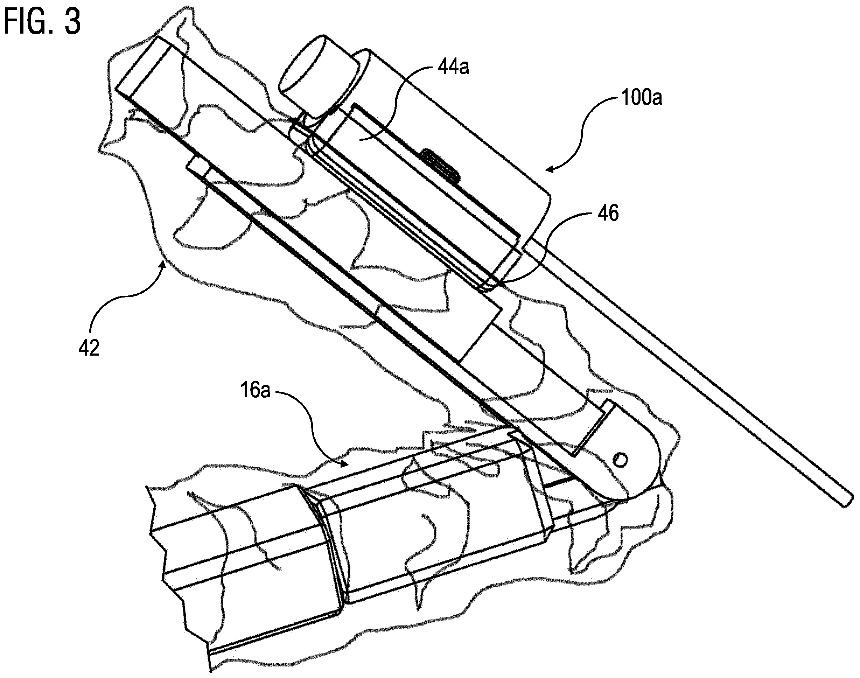

Figur 3 zeigt den Teil des Manipulatorarms 16a nach Figur 2 mit der Koppeleinheit 44a sowie die Sterilbarriere 42 und die Instrumenteneinheit 100a in einem gekoppelten Zustand. Im gekoppelten Zustand ist die Sterilbarriere 42, insbesondere der formstabile Teil 46, über die Koppeleinheit 44a und den Manipulatorarm 16a gezogen. Anschließend wurde die Instrumenteneinheit 100a zu einem Verbindungsbereich der Koppeleinheit 44a geführt, so dass der Verbindungsbereich der Koppeleinheit 44a und ein Verbindungsbereich der Instrumenteneinheit 100a, von der Sterilbarriere 42 getrennt, einander gegenüberliegend angeordnet sind. Die Instrumenteneinheit 100a kann reversibel mit Hilfe von Laschen an der Koppeleinheit 44a befestigt werden. Alternativ oder zusätzlich ist eine Befestigung mit Hilfe von Magneten oder einer anderen geeigneten Befestigung der Instrumenteneinheit 100a an der Koppeleinheit 44a möglich.

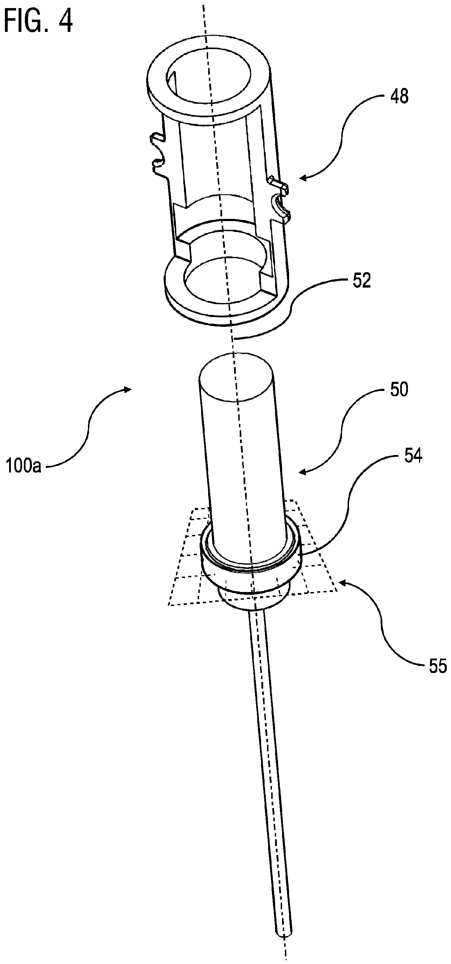

Figur 4 zeigt eine schematische Explosionszeichnung der Instrumenteneinheit 100a mit einem Außenteil 48 und einem Innenteil 50. Das Innenteil 50 ist in einem zusammengebauten Zustand der Instrumenteneinheit 100a innerhalb des Außenteils 48 drehbar gelagert. Dabei ist das Innenteil 50 um eine Rotationsachse 52 relativ zu dem Außenteil 48 rotierbar, insbesondere ist das Instrumentenantriebselement 54 um die Rotationsachse 52 relativ zu dem Außenteil 48 rotierbar. Das Instrumentenantriebselement 54 ist außerdem in einer Rotationsebene 55 angeordnet und rotiert in dieser Ebene 55. Die Ebene 55 ist orthogonal zu der Rotationsachse 52. Das Außenteil 48 ist im gekoppelten Zustand ortsfest mit der Koppeleinheit 44a verbunden, wohingegen das Innenteil 50 relativ zum Außenteil 48 frei drehbar ist.

Alternativ kann die Instrumenteneinheit 100a nur das Innenteil 50 umfassen und das Innenteil 50 in der Koppeleinheit 44a drehbar gelagert sein. In einer weiteren Alternative kann die Instrumenteneinheit 100a nur das Innenteil 50 umfassen, wobei das Innenteil 50 drehfest mit der Koppeleinheit 44a verbunden ist und das Instrumentenantriebselement 54 der Instrumenteneinheit 100a innerhalb des Innenteils 50 drehbar gelagert ist.

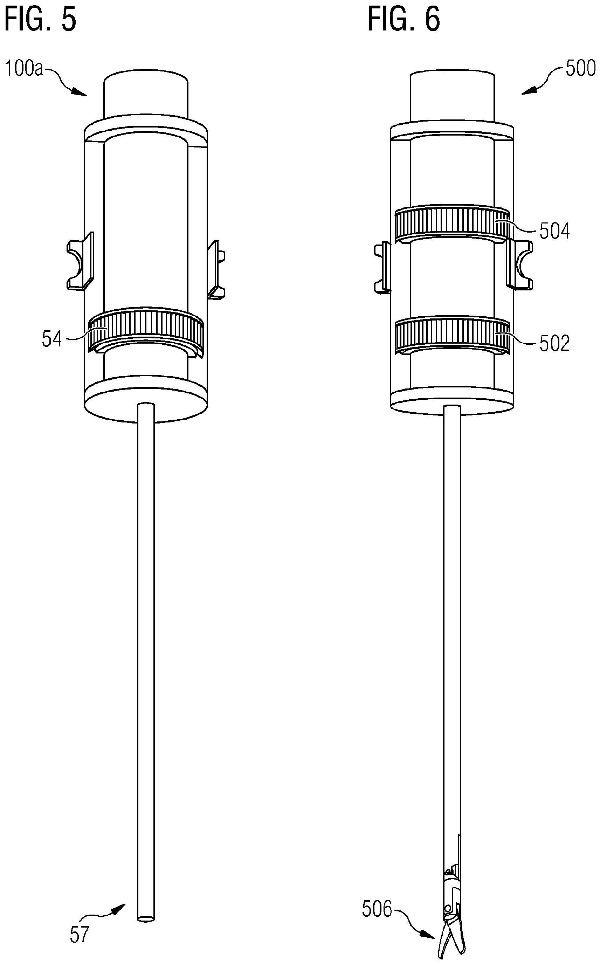

Figur 5 zeigt eine Seitenansicht der Instrumenteneinheit 100a mit dem Instrumentenantriebselement 54 in einem zusammengebauten Zustand. Mit Hilfe des Instrumentenantriebselements 54 kann ein Endeffektor mit einem Freiheitsgrad bewegt und/oder gesteuert werden. Das Instrumentenantriebselement 54 ist mit einem Endeffektor mechanisch verbunden, sodass durch Rotation des Instrumentenantriebselements 54 der Endeffektor bewegt und/oder gesteuert werden kann. Der Endeffektor kann mit Hilfe eines Getriebes mit dem Instrumentenantriebselement 54 verbunden sein. Beispielsweise kann so eine Drehbewegung des Instrumentenantriebselements 54 in eine Linearbewegung entlang der Rotationsachse 52 umgesetzt werden. Vorzugsweise ist der Endeffektor an einem Ende 57 eines Schafts der Instrumenteneinheit 100a angeordnet. Im Ausführungsbeispiel ist der Endeffektor eine Optik eines Endoskops und hat einen Freiheitsgrad. Mit Hilfe des Instrumentenantriebselements 54 kann die Optik des Endoskops und/oder der Bilderfassungssensor/die Bilderfassungssensoren des Endoskops beispielsweise gedreht oder geschwenkt werden. Vorzugsweise wird hierzu das gesamte Endoskop gedreht.

Alternativ kann der Endeffektor ein chirurgisches Instrument sein, wie beispielsweise eine Schere, ein Nadelhalter, eine Klammer oder eine Zange.

Figur 6 zeigt eine Instrumenteneinheit 500 gemäß einer zweiten Ausführungsform mit einem ersten Instrumentenantriebselement 502 und einem zweiten Instrumentenantriebselement 504. In diesem Fall kann ein Endeffektor in zwei Freiheitsgraden bewegbar und/oder steuerbar sein. Der Endeffektor der Instrumenteneinheit 500 ist eine Schere 506, die beispielsweise geöffnet und geschlossen werden kann. In Figur 6 ist die Schere 506 in einem geöffneten Zustand gezeigt.

Alternativ kann der Endeffektor beispielsweise eine Optik eines Endoskops sein, die sowohl um die Rotationsachse 52 drehbar ist als auch mit Hilfe eines Gelenks um eine Achse orthogonal zur Rotationsachse 52 bewegbar ist.

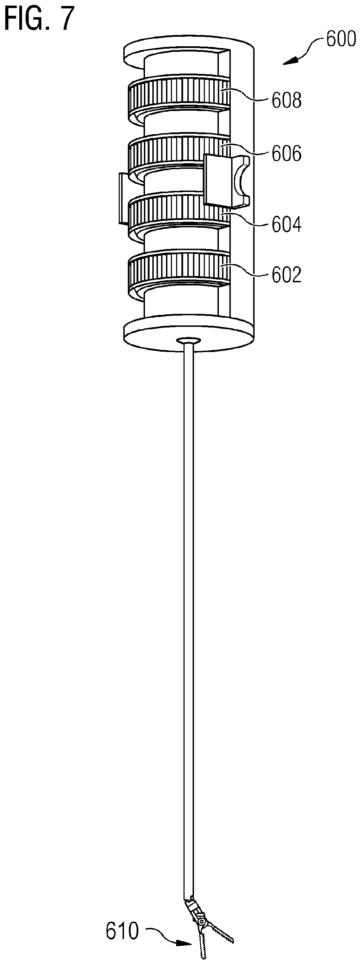

Figur 7 zeigt eine Instrumenteneinheit 600 gemäß einer dritten Ausführungsform mit insgesamt vier Instrumentenantriebselementen 602, 604, 606, 608. In diesem Fall kann ein Endeffektor in vier Freiheitsgraden bewegbar und/oder steuerbar sein. Beispielsweise kann der Endeffektor eine Schere oder Zange sein. Der Endeffektor der Instrumenteneinheit 600 ist eine Zange 610. In diesem Fall sind beispielsweise sowohl die Hebel beziehungsweise Schenkel der Zange 610 relativ zueinander bewegbar als auch die Zange 610 um eine X-Achse, Y-Achse und/oder Z-Achse drehbar beziehungsweise kippbar. Das erlaubt eine einfache und genaue Positionierung des Endeffektors relativ zum Operationsfeld. In Figur 7 ist die Zange 610 in einem geöffneten Zustand, sowie aus der Achse 52 gekippt gezeigt.

Die Rotationsachse 52 verläuft durch die Kreismittelpunkte der vier Instrumentenantriebselemente 602, 604, 606, 608 der Instrumenteneinheit 600. Die Instrumentenantriebselemente 602, 604, 606, 608 sind in parallelen zur Rotationsachse 52 orthogonal verlaufenden Rotationsebenen angeordnet.

Jede der Instrumenteneinheiten 100a, 500, 600 kann jeweils ein Identifizierungselement zugeordnet sein, das von einer Ausleseeinheit in der Koppeleinheit 44a ausgelesen werden kann. Beispielsweise kann eine Instrumenteneinheit 100a, 500, 600 ein RFID-Element umfassen, das berührungslos mit Hilfe der Koppeleinheit 44a ausgelesen werden kann. Das Identifizierungselement kann Informationen über die Instrumenteneinheit 100a, 500, 600 umfassen, wie beispielsweise Anzahl der Freiheitsgrade und Anzahl der Instrumentenantriebselemente, Bewegungsspielraum der einzelnen Instrumentenantriebselemente und Art des Endeffektors. Zusätzlich können Informationen über den Zustand der Instrumenteneinheit 100a, 500, 600 übermittelt werden, insbesondere ob die Instrumenteneinheit 100a, 500, 600 unbenutzt und steril ist, um eine Nutzung von verunreinigten Instrumenteneinheiten 100a, 500, 600 zu verhindern.

Darüber hinaus können weitere Schnittstellen zwischen der Koppeleinheit 44a und der Instrumenteneinheit 100a, 500, 600 zur Informationsübertragung vorgesehen sein. Vorzugsweise sind die Schnittstellen berührungslos, beispielsweise optische Schnittstellen und/oder Schnittstellen basierend auf anderen elektromagnetischen Wellen.

Bei anderen Ausführungsformen können bei den Instrumenteneinheiten 500 und 600 auch andere Endeffektoren eingesetzt und angetrieben werden.

Im Folgenden werden alternative Antriebsarten beschrieben, um ein Instrumentenantriebselement einer Instrumenteneinheit mit Hilfe einer Koppeleinheit anzutreiben.

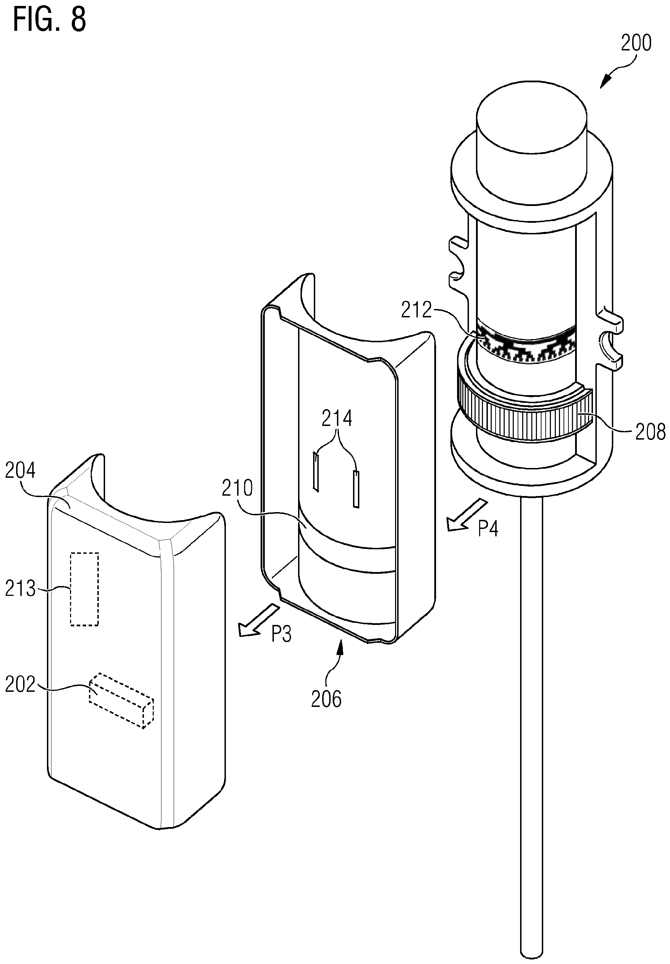

Figuren 8 bis 10 zeigen jeweils eine Instrumenteneinheit 200 und eine Koppeleinheit 204 gemäß einer vierten Ausführungsform mit einem elektromagnetischen Antrieb. Figur 8 zeigt ein Koppeleinheitsantriebselement 202 in der Koppeleinheit 204. Ein Teil 206 der Sterilbarriere 42 wird in Richtung des Pfeils P3 über die Koppeleinheit 204 gestülpt. Anschließend wird die Instrumenteneinheit 200 in Richtung des Pfeils P4 an die Koppeleinheit 204 angekoppelt.

Die Instrumenteneinheit 200 umfasst ein Instrumentenantriebselement 208. Im gekoppelten Zustand sind das Koppeleinheitsantriebselement 202 und das Instrumentenantriebselement 208 in der Rotationsebene 55 orthogonal zur Rotationsachse 52 nebeneinander angeordnet. Der formstabile Teil 206 der Sterilbarriere 42 ist so ausgebildet und angeordnet, dass in einem Bereich 210 entlang der Rotationsebene 55 der Antriebselemente 202, 208 der formstabile Teil 206 der Sterilbarriere 42 eng an dem Koppeleinheitsantriebselement 202 anliegt und nicht in Kontakt mit dem Instrumentenantriebselement 208 kommt. Insbesondere ist der formstabile Teil 206, das Koppeleinheitsantriebselement 202 und das Instrumentenantriebselement 208 jeweils so ausgebildet und zueinander angeordnet, dass der Abstand zwischen dem Koppeleinheitsantriebselement 202 und dem Instrumentenantriebselement 208 im gekoppelten Zustand 0,05 mm bis 2 mm ist und der formstabile Teil 206 das rotierende Instrumentenantriebselement 208 nicht berührt.

Zusätzlich umfasst die Instrumenteneinheit 200 ein optisch erfassbares Muster 212, das den Instrumentenantriebseinheiten 208 zugeordnet ist und bei einer Rotation des Instrumentenantriebselements 208 zusammen mit diesem um die Rotationsachse 52 rotiert. Das Muster 212 wird im gekoppelten Zustand der Koppeleinheit 204 und der Instrumenteneinheit 200 mit Hilfe eines oder mehrerer optischer Sensoren des Koppeleinheitsantriebselements 202 erfasst, wobei ein erfassbarer Drehwinkel des Instrumentenantriebselements 208 um die Rotationsachse 52 von einer Leseeinheit 213 berührungslos ermittelt wird. Der erfassbare Drehwinkel wird dabei von einer Auflösung des optischen Sensors und einer Auflösung des Musters bestimmt. Das Muster 212 ist so ausgebildet und um den Umfang des Instrumentenantriebselements 208 angeordnet, dass der optische Sensor für jeden erfassbaren Drehwinkel einen eindeutig unterscheidbaren Teil des Musters 212 erfasst. Dieser Teil des Musters 212 ist von anderen bei weiteren Drehwinkeln erfassbaren Teilen des Musters 212 verschieden. Das Muster 212 kann insbesondere ein sogenannter Gray-Code sein. Somit ist eine eindeutige Positionsbestimmung des Instrumentenantriebselements 208 um die Rotationsachse 52 möglich. Um eine zuverlässige optische Erfassung des Musters 212 zu gewährleisten sind in dem Teil 206 zumindest einzelne Bereiche 214 aus optisch transparentem Material vorgesehen.

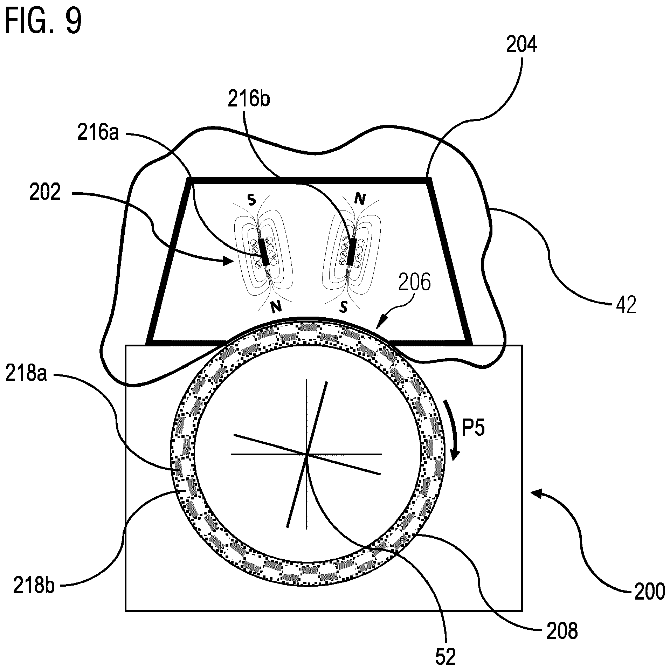

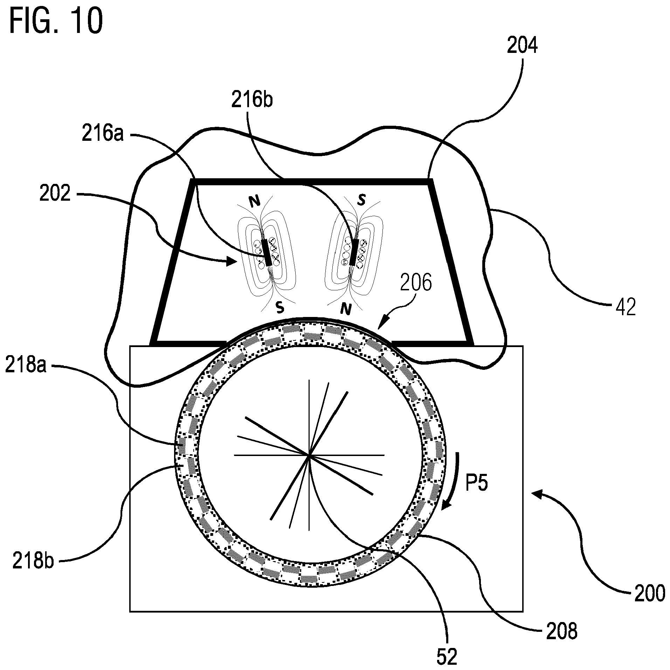

Das Koppeleinheitsantriebselement 202 und das Instrumentenantriebselement 208 umfassen im Ausführungsbeispiel nach Figur 8 magnetische und/oder magnetfelderzeugende Elemente. Figuren 9 und 10 zeigen schematische Schnittzeichnungen des elektromagnetischen Antriebsprinzips nach Figur 8, wobei die Schnittebene die Rotationsebene 55 ist. Die Koppeleinheit 204 umfasst als Teil des Koppeleinheitsantriebselements 202 mehrere magnetfelderzeugende Elemente 216a, 216b. Diese Elemente 216a, 216b sind beispielsweise Elektromagnete. In den schematischen Zeichnungen in Figuren 9 und 10 sind jeweils zwei magnetfelderzeugenden Elemente 216a, 216b dargestellt, alternativ sind eine größere Anzahl an Elementen 216a, 216b möglich. Die Elemente 216a, 216b sind in der Rotationsebene 55 um einen Teil des Umfangs des Instrumentenantriebselements 208 angeordnet. Das Koppeleinheitsantriebselement 202 umfasst das Instrumentenantriebselement 208 in einem Winkel von 45° bis 180° um die Rotationsachse. Die Polung der magnetischen und magnetfelderzeugenden Elemente ist in Figur 8 bis 10 exemplarisch mit N (Nordpol) oder S (Südpol) gekennzeichnet.

Das Instrumentenantriebselement 208 umfasst entlang des Umfangs des Antriebselements 208 eine Vielzahl von Permanentmagneten 218a, 218b. Diese Magnete 218a, 218b haben eine abwechselnd entgegengesetzte Polarisierung. Die Bewegung des Instrumentenantriebselements 208 wird durch das Koppeleinheitsantriebselement 202 nach dem Prinzip eines Elektromotors, insbesondere eines Schrittmotors, erzeugt. Dabei ist das Koppeleinheitsantriebselement 202 der Stator und das Instrumentenantriebselement 208 der Rotor. Die magnetfelderzeugenden Elemente 216a, 216b sind nicht bewegbar, das heißt ortsfest, in dem Koppeleinheitsantriebselement 202 angeordnet. Im Ausführungsbeispiel nach Figuren 8 bis 10 hat das Instrumentenantriebselement 208 eine zylindrische Form. Alternativ kann die Mantelfläche lediglich im Wesentlichen zylindrisch sein und beispielsweise eine Form mit Grundfläche eines regelmäßigen Polygons mit Zentrum in der Rotationsachse 52 sein. Zumindest eine dieses Polygon einschließende Hüllmantelfläche ist dann zylindrisch.

Mit Hilfe der magnetfelderzeugenden Elemente 216a, 216b erzeugt das Koppeleinheitsantriebselement 202 ein elektromagnetisches Feld. Die Elemente 216a, 216b werden so angesteuert, dass sie mit jedem Schritt ihren Zustand ändern. Figur 9 zeigt dabei einen ersten Zustand mit einer ersten Polarisierung der magnetfelderzeugenden Elemente 216a, 216b. Figur 10 zeigt einen zweiten Zustand mit einer zweiten, jeweils umgekehrten Polarisierung der magnetfelderzeugenden Elemente 216a, 216b. Dadurch erzeugt das Koppeleinheitsantriebselement 202 ein sich schrittweise bewegendes elektromagnetisches Feld. Das erzeugte elektromagnetische Feld wirkt mit einer Kraft auf die magnetischen Elemente 218a, 218b des Instrumentenantriebselements 208. Durch jeden Wechsel der Polarisierung der Elemente 216a, 216b wird somit das Instrumentenantriebselement 208 durch die Magnetkraft um einen Winkel in Richtung des Pfeils P5 um die Rotationsachse 52 gedreht. Die Drehung des Instrumentenantriebselements 208 kann mit Hilfe von Getrieben in eine gewünschte Bewegung und/oder Steuerung des Endeffektors umgesetzt werden.

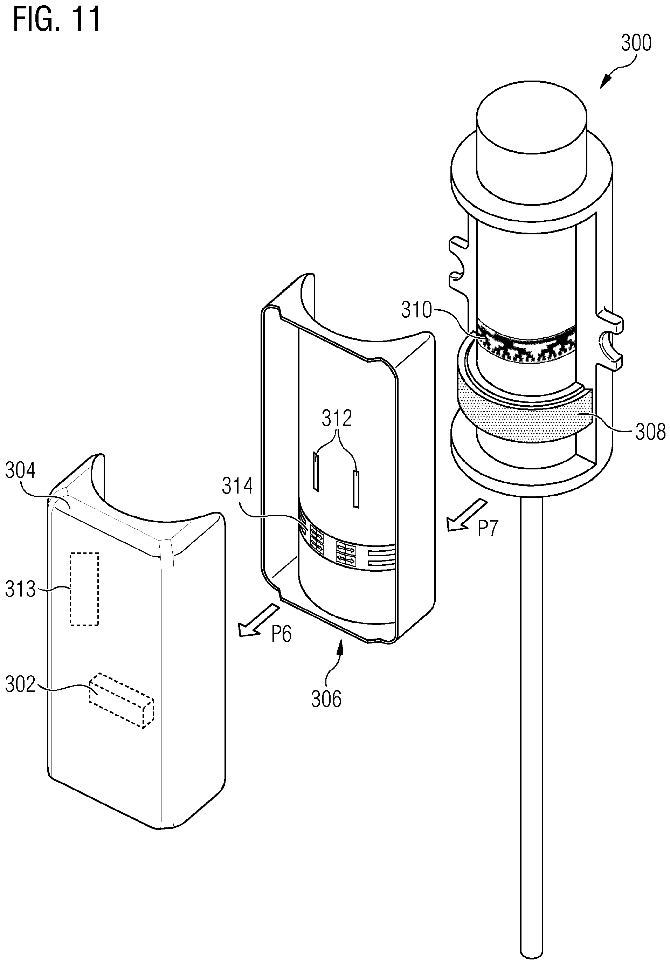

Figuren 11 bis 13 zeigen eine Instrumenteneinheit 300 und eine Koppeleinheit 304 gemäß einer fünften Ausführungsform mit einem Antrieb mit einer mechanischen Kraftübertragung. Die mechanische Kraftübertragung wird insbesondere mit Hilfe von Kraft- und/oder Formschluss erreicht. Der Antrieb kann beispielsweise als Piezomotor ausgebildet sein. Alternativ kann die mechanische Kraftübertragung mit Hilfe von Nockenwellen oder von Koppelgetrieben erfolgen.

Figur 11 zeigt ein Koppeleinheitsantriebselement 302 in der Koppeleinheit 304. Ein formstabiler Teil 306 der Sterilbarriere 42 wird in Richtung des Pfeils P6 über die Koppeleinheit 304 gestülpt. Anschließend kann die Instrumenteneinheit 300 in Richtung des Pfeils P7 an die Koppeleinheit 304 angekoppelt werden. Die Instrumenteneinheit 300 umfasst ein Instrumentenantriebselement 308. Im gekoppelten Zustand sind das Koppeleinheitsantriebselement 302 und das Instrumentenantriebselement 308 entlang der Rotationsebene 55 orthogonal zur Rotationsachse 52 nebeneinander angeordnet.

Die Instrumenteneinheit 300 umfasst ein optisch erfassbares Muster 310. Dieses Muster 310 wird im gekoppelten Zustand mit Hilfe eines oder mehrerer optischer Sensoren des Koppeleinheitsantriebselement 302 erfasst und damit ein erfassbarer Drehwinkel des Instrumentenantriebselements 308 um die Rotationsachse 52 von einer Leseeinheit 313 berührungslos ermittelt. Wie weiter oben für Instrumenteneinheit 200 beschrieben, ist das Muster 310 so ausgebildet und um den Umfang des Instrumentenantriebselements 308 angeordnet, dass der optische Sensor für jeden erfassbaren Drehwinkel einen eindeutig unterscheidbaren Teil des Musters 310 erfasst. Das Muster 310 kann insbesondere ein sogenannter Gray-Code sein. Somit ist eine eindeutige Positionsbestimmung des Instrumentenantriebselements 308 um die Rotationsachse 52 möglich. Um eine zuverlässige optische Erfassung des Musters 310 zu gewährleisten sind in dem formstabilen Teil 306 Bereiche 312 aus optisch transparentem Material vorgesehen.

Das Koppeleinheitsantriebselement 302 hat im Ausführungsbeispiel nach Figur 11 bewegliche Eingriffselemente, die im gekoppelten Zustand der Instrumenteneinheit 300 mit der Koppeleinheit 304 zumindest zeitweise im Eingriff mit der Mantelfläche des Instrumentenantriebselements 308 sind. Um die Integrität der Sterilbarriere 42 zwischen den Antriebselementen 302, 308, insbesondere des formstabilen Teils 306, sicherzustellen, ist im formstabilen Teil 306 ein flexibler Bereich 314 vorgesehen der so ausgebildet ist, dass er eine Bewegung und/oder Verformung der Eingriffselemente ermöglicht, ohne beschädigt zu werden.

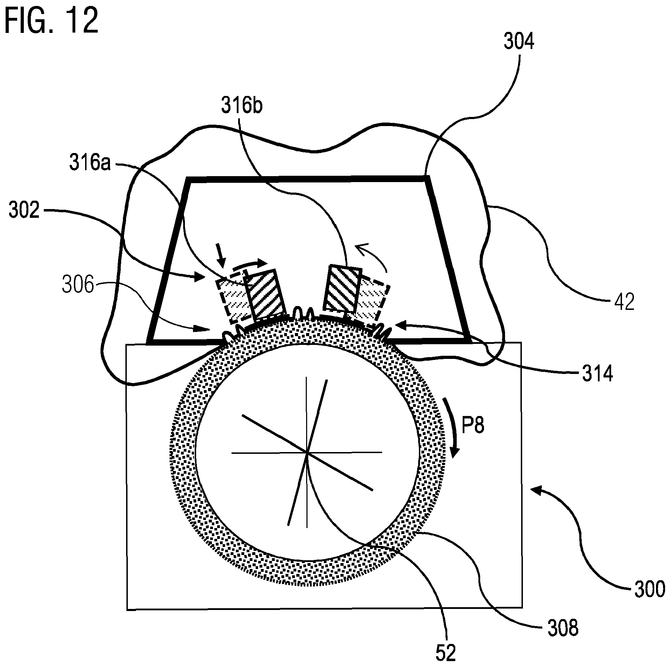

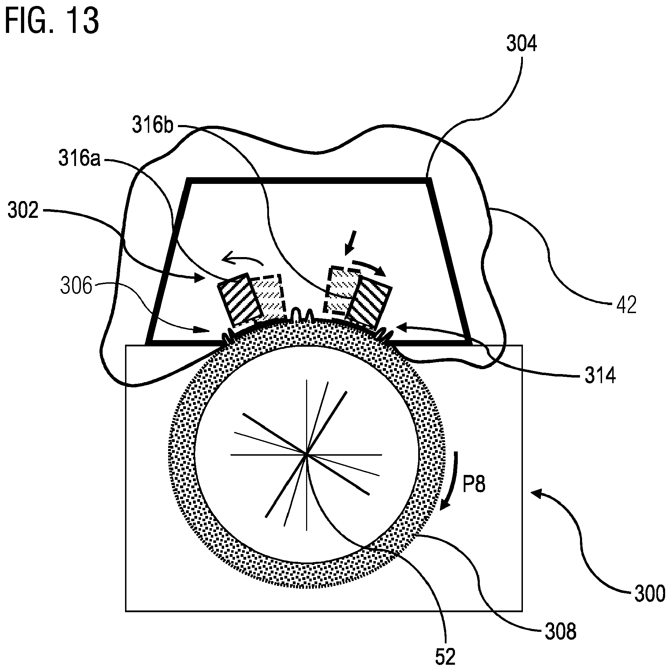

Figuren 12 und 13 zeigen schematische Schnittzeichnungen des mechanischen Antriebsprinzips nach Figur 11, wobei die Schnittebene die Rotationsebene 55 ist. Die Koppeleinheit 304 umfasst das Koppeleinheitsantriebselement 302 mit mehreren Eingriffselementen 316a, 316b. Diese Elemente 316a, 316b sind bewegbar und/oder verformbar und zumindest zeitweise in Eingriff mit der Mantelfläche des Instrumentenantriebselements 308. In den schematischen Zeichnungen nach Figur 12 und 13 sind zwei Eingriffselemente 316a, 316b dargestellt, alternativ sind eine größere Anzahl an Eingriffselementen 316a, 316b möglich. Die Eingriffselemente 316a, 316b sind in der Rotationsebene 55 um einen Teil des Umfangs des Instrumentenantriebselements 308 angeordnet. Weiterhin sind die Eingriffselemente 316a, 316b in der Rotationsebene 55 bewegbar und/oder verformbar.

Die Oberfläche der Mantelfläche des Instrumentenantriebselements 308 ist so ausgebildet, dass die Eingriffselemente 316a, 316b mit der Mantelfläche einen Kraft- oder Formschluss bilden können. Im Ausführungsbeispiel nach Figuren 11 bis 13 hat das Instrumentenantriebselement 308 eine zylindrische Form, wobei die Mantelfläche parallel zu der Seite des Koppeleinheitsantriebselements 302 ausgerichtet ist, die im gekoppelten Zustand auf das Instrumentenantriebselement 308 gerichtet ist. Alternativ kann die Mantelfläche lediglich im Wesentlichen zylindrisch sein und/oder im Wesentlichen parallel zum Koppeleinheitsantriebselement 302 ausgerichtet sein. Beispielsweise ist die Oberfläche der Mantelfläche des Instrumentenantriebselements 308 aufgeraut oder hat einen Zahnkranz, um einen sicheren Kraft- und/oder Formschluss mit dem Eingriffselement zu ermöglichen.

Um das Instrumentenantriebselement 308 mit Hilfe des Koppeleinheitsantriebselements 302 um die Rotationsachse 52 zu drehen, ist jeweils ein Eingriffselement 316a, 316b in Form- oder Kraftschluss mit dem Instrumentenantriebselement 308. Durch Bewegen des Eingriffselements 316a, 316b um die Rotationsachse 52 dreht sich das Instrumentenantriebselement 308 um die Rotationsachse 52 in Richtung des Pfeils P8. Die Drehung des Instrumentenantriebselements 308 kann mit Hilfe von Getrieben in eine Bewegung und/oder Steuerung des Endeffektors übersetzt werden. In Figur 12 bewegt sich das Eingriffselement 316a in einem ersten Schritt in Richtung des Instrumentenantriebselements 308, tritt in eine kraftschlüssige Verbindung mit dem Instrumentenantriebselement 308 und dreht anschließend das Instrumentenantriebselement 308 in Richtung des Pfeils P8. Gleichzeitig löst sich das Eingriffselement 316b von dem Instrumentenantriebselement 308 und bewegt sich entgegen der Drehrichtung P8 in eine Ausgangslage zurück. In Figur 13 bewegt sich das Eingriffselement 316b in einem zweiten Schritt in Richtung des Instrumentenantriebselements 308, tritt in eine kraftschlüssige Verbindung mit dem Instrumentenantriebselement 308 und dreht das Instrumentenantriebselement 308 in Richtung des Pfeils P8. Analog zu Figur 12 löst sich währenddessen das Eingriffselement 316a von dem Instrumentenantriebselement 308 und bewegt sich entgegen der Drehrichtung P8 in eine Ausgangslage zurück. Vorzugsweise ist immer mindestens ein Eingriffselement 316a, 316b in Kraft- und/oder Formschluss mit dem Instrumentenantriebselement 308, sodass das Instrumentenantriebselement 308 gegen ein ungewolltes Drehen gesichert ist.

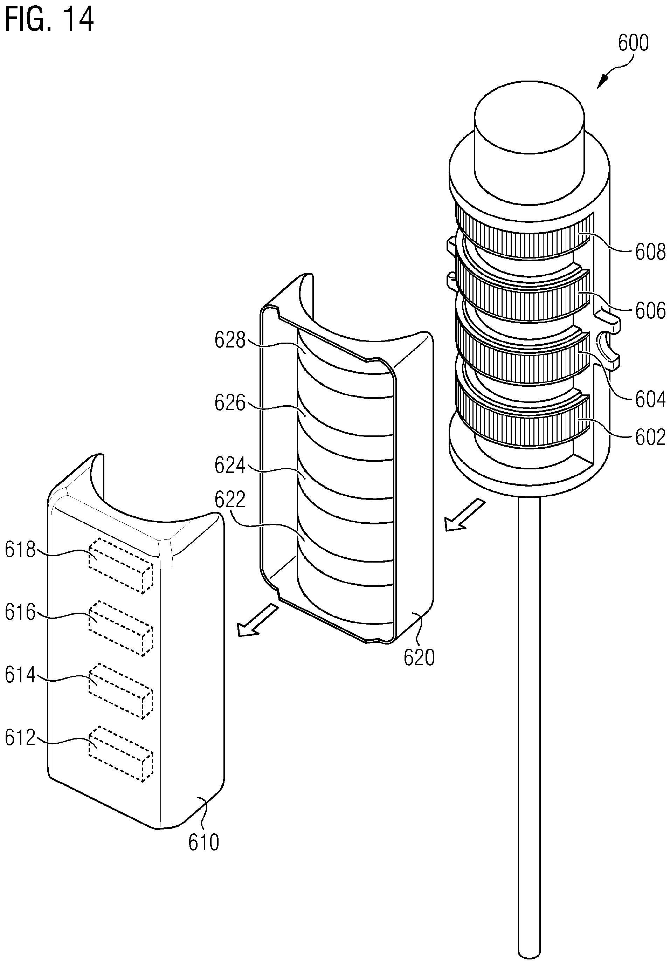

Figur 14 zeigt die Instrumenteneinheit 600 gemäß der Ausführungsform nach Figur 7 und eine Koppeleinheit 610. Die Instrumenteneinheit 600 umfasst vier Instrumentenantriebselemente 602, 604, 606, 608. Die Koppeleinheit 610 umfasst vier Koppeleinheitsantriebselement 612, 614, 616, 618. Die Koppeleinheitsantriebselemente 612, 614, 616, 618 sind übereinander jeweils in einer Rotationsebene eines Instrumentenantriebselements 602, 604, 606, 608 angeordnet. Somit sind die Koppeleinheitsantriebselemente 612, 614, 616, 618 in einer Linie parallel zu der Rotationsachse 52 der Instrumenteneinheit 600 angeordnet. Weiterhin ist jedes Koppeleinheitsantriebselement 612, 614, 616, 618 zumindest entlang eines Teils der Hüllmantelfläche eines Instrumentenantriebselements 602, 604, 606, 608 gegenüberliegend angeordnet.

Jedes Instrumentenantriebselement 602, 604, 606, 608 kann ein umlaufendes Muster zugeordnet sein, um den Drehwinkel des Instrumentenantriebselement 602, 604, 606, 608 um die Rotationsachse 52, nach der weiter oben beschriebenen Methode, zu erfassen. Außerdem kann ein formstabiler Teil 620 einer Sterilbarriere einen optisch transparenten Bereich umfassen, um das Muster mit Hilfe eines optischen Sensors erfassen zu können.

Der formstabile Teil 620 der Sterilbarriere umfasst weiterhin mehrere Bereiche 622, 624, 626, 628 die so ausgebildet sind, dass sie je nach Antriebsart entweder eine Bewegung und/oder Verformung durch Eingriffselemente ermöglichen ohne beschädigt zu werden oder dass sie eng an den Koppeleinheitsantriebselementen 612, 614, 616, 618 anliegen und nicht in Kontakt mit den Instrumentenantriebselementen 602, 604, 606, 608 kommen. Im Falle eines Antriebs nach der fünften Ausführungsform in Figuren 11 bis 13 ist zumindest ein zeitweiser Kontakt der Bereiche 622, 624, 626, 628 vorgesehen, wenn ein Eingriffselement mit einem Instrumentenantriebselement 602, 604, 606, 608 in Kraft- und/oder Formschluss ist.

Die Anzahl der Koppeleinheitsantriebselemente 612, 614, 616, 618 und Instrumentenantriebselemente 602, 604, 606, 608 kann in anderen Ausführungsformen abweichen, wie in den vorherigen Ausführungsbeispielen weiter oben beschrieben. Mindestens sind jeweils ein Koppeleinheitsantriebselement und ein Instrumentenantriebselement vorgesehen. Darüber hinaus umfasst die Koppeleinheit mindestens so viele Koppeleinheitsantriebselemente wie die Anzahl an Instrumentenantriebselementen der Instrumenteneinheit. Somit ist im gekoppelten Zustand jedes Koppeleinheitsantriebselement mit einem Instrumentenantriebselement in einer Rotationsebene 55 paarweise angeordnet.

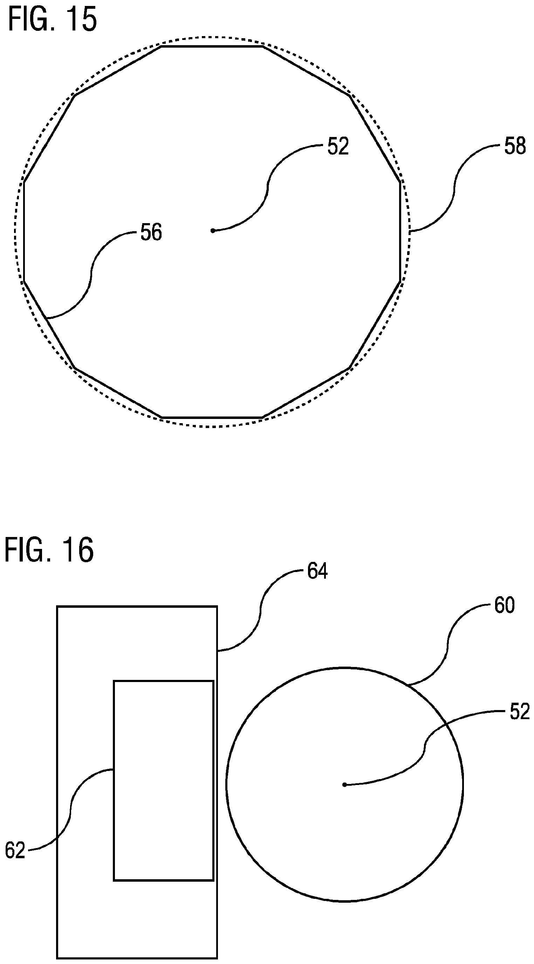

Figur 15 zeigt eine schematische Schnittzeichnung eines Instrumentenantriebselements 56 gemäß einer sechsten Ausführungsform einer Instrumenteneinheit, wobei die Schnittebene die Rotationsebene 55 ist. Das Instrumentenantriebselement 56 hat eine Form mit der Grundfläche eines regelmäßigen 12-eckigen Polygons und einem Zentrum in der Rotationsachse 52. Eine angenommene Hüllmantelfläche 58 hat dabei eine zylindrische Form mit Mittelpunkt auf der Rotationsachse 52 und einem Radius der so gewählt ist, dass die Hüllmantelfläche 58 das Polygon genau umschließt.

Figur 16 zeigt eine verallgemeinerte schematische Darstellung des Antriebs einer Instrumenteneinheit als Schnittzeichnung entlang der Rotationsebene 55. Dabei wird ein Instrumentenantriebselement 60 mit Hilfe eines Koppeleinheitsantriebselements 62 um die Rotationsachse 52 gedreht. Eine Sterilbarriere 64 umschließt das Koppeleinheitsantriebselement 62. Insbesondere ist die Sterilbarriere 64 zwischen dem Koppeleinheitsantriebselement 62 und dem Instrumentenantriebselements 60 durchgehend angeordnet.

### Bezugszeichenliste

- 10: Vorrichtung zur robotergestützten Chirurgie
- 12: Manipulator
- 14: Stativ
- 16a bis 16d: Manipulatorarm
- 20: Stativkopf
- 22: Stativarmantriebseinheit
- 24: Stativfuß
- 26: Stativfußantriebseinheit
- 28, 30: Stativarm
- 32: Operationstischsäule
- 34: Operationstisch
- 36: Steuereinheit
- 38: Ein- und Ausgabeeinheit
- 40: steriler Operationsbereich
- 42, 64: Sterilbarriere
- 44a, 204, 304, 610: Koppeleinheit
- 46, 206, 306, 620: formstabiler Teil der Sterilbarriere
- 48: Außenteil der Instrumenteneinheit
- 50: Innenteil der Instrumenteneinheit
- 52: Rotationsachse
- 54, 60, 208, 308, 502, 504, 602, 604, 606, 608: Instrumentenantriebselement
- 55: Rotationsebene
- 56: polygonförmiges Instrumentenantriebselement
- 57: Schaftende
- 58: Hüllmantelfläche
- 62, 202, 302, 612, 614, 616, 618: Koppeleinheitsantriebselement
- 100a bis 100d, 200, 300, 500, 600: Instrumenteneinheit
- 210, 622, 624, 626, 628: Bereich der Sterilbarriere
- 212, 310: Muster
- 214, 312: optisch transparenter Bereich
- 213, 313: Leseeinheit
- 216a, 216b: magnetfelderzeugendes Element
- 218a, 218b: Permanentmagnet
- 314: flexibler Bereich der Sterilbarriere
- 316a, 316b: Eingriffselement
- 506, 610: Endeffektor

## Patentansprüche

1. Vorrichtung zur robotergestützten Chirurgie,
mit mindestens einem Manipulatorarm (16a bis 16d) mit einer nicht sterilen Koppeleinheit (44a, 204, 304, 610), die mindestens ein erstes Antriebselement (62, 202, 302, 612, 614, 616, 618) umfasst,
mit einer in einem sterilen Bereich (40) angeordneten sterilen Instrumenteneinheit (100a bis 100d, 200, 300, 500, 600), die mindestens ein zweites Antriebselement (54, 60, 208, 308, 502, 504, 602, 604, 606, 608) umfasst, das um eine Rotationsachse (52) rotierbar angeordnet ist,
wobei die Instrumenteneinheit (100a bis 100d, 200, 300, 500, 600) mit der Koppeleinheit (44a, 204, 304, 610) des Manipulatorarms (16a bis 16d) koppelbar ist,
wobei das erste Antriebselement (62, 202, 302, 612, 614, 616, 618) und das zweite Antriebselement (54, 60, 208, 308, 502, 504, 602, 604, 606, 608) derart ausgebildet und in einem gekoppelten Zustand derart angeordnet sind, dass durch das erste Antriebselement (62, 202, 302, 612, 614, 616, 618) eine Kraft auf das zweite Antriebselement (54, 60, 208, 308, 502, 504, 602, 604, 606, 608), zur Rotation des zweiten Antriebselements (54, 60, 208, 308, 502, 504, 602, 604, 606, 608) um die Rotationsachse (52), ausübbar ist,
wobei im gekoppelten Zustand das erste Antriebselement (62, 202, 302, 612, 614, 616, 618) und das zweite Antriebselement (54, 60, 208, 308, 502, 504, 602, 604, 606, 608) in einer Rotationsebene (55) senkrecht zur Rotationsachse (52) nebeneinander angeordnet sind, und
mit einer Sterilbarriere (42,64), die zumindest zwischen dem ersten Antriebselement (62, 202, 302, 612, 614, 616, 618) und dem zweiten Antriebselement (54, 60, 208, 308, 502, 504, 602, 604, 606, 608) angeordnet ist.

2. Vorrichtung nach Anspruch 1, **dadurch gekennzeichnet, dass** im gekoppelten Zustand das erste Antriebselement (62, 202, 302, 612, 614, 616, 618) in der Rotationsebene des zweiten Antriebselements (54, 60, 208, 308, 502, 504, 602, 604, 606, 608) so angeordnet ist, dass eine dem zweiten Antriebselement (54, 60, 208, 308, 502, 504, 602, 604, 606, 608) zugewandte Oberfläche des ersten Antriebselements (62, 202, 302, 612, 614, 616, 618) in zumindest einem Bereich parallel zu einer zylindrischen Mantelfläche oder einer zylindrischen Hüllmantelfläche des zweiten Antriebselements (54, 60, 208, 308, 502, 504, 602, 604, 606, 608) verläuft.

3. Vorrichtung nach Anspruch 1 oder 2, **dadurch gekennzeichnet, dass** in der Rotationsebene an einer dem zweiten Antriebselement (54, 60, 208, 308, 502, 504, 602, 604, 606, 608) zugewandten Seite des ersten Antriebselements (62, 202, 302, 612, 614, 616, 618) magnetfelderzeugende Elemente (216a, 216b) angeordnet sind und auf einer Kreisbahn um die Rotationsachse magnetfelderzeugende oder magnetische Elemente (218a, 218b) des zweiten Antriebselements (54, 60, 208, 308, 502, 504, 602, 604, 606, 608) angeordnet sind.

4. Vorrichtung nach Anspruch 3, **dadurch gekennzeichnet, dass** die magnetfelderzeugenden Elemente (216a, 216b) des ersten Antriebselements (62, 202, 302, 612, 614, 616, 618) Elektromagnete sind und die magnetfelderzeugenden Elemente (218a, 218b) des zweiten Antriebselements (54, 60, 208, 308, 502, 504, 602, 604, 606, 608) Elektromagnete mit umlaufend abwechselnden Polen sind, oder
dass die magnetfelderzeugenden Elemente (216a, 216b) des ersten Antriebselements (62, 202, 302, 612, 614, 616, 618) Elektromagnete sind und die magnetischen Elemente (218a, 218b) des zweiten Antriebselements (54, 60, 208, 308, 502, 504, 602, 604, 606, 608) Permanentmagnete mit umlaufend abwechselnden Polen sind.

5. Vorrichtung nach Anspruch 4, **dadurch gekennzeichnet, dass** in dem ersten Antriebselement (62, 202, 302, 612, 614, 616, 618) mit Hilfe der Elektromagnete (216a, 216b) des ersten Antriebselements (62, 202, 302, 612, 614, 616, 618) Magnetfelder erzeugbar sind, durch die die Kraft auf die Permanentmagnete (218a, 218b) oder die Elektromagnete des zweiten Antriebselements (54, 60, 208, 308, 502, 504, 602, 604, 606, 608) ausübbar ist, wobei durch Änderung der Polung der Elektromagnete (216a, 216b) des ersten Antriebselements (62, 202, 302, 612, 614, 616, 618) und/oder Änderung eines Schaltzustands der Elektromagnete (216a, 216b) des ersten Antriebselements (62, 202, 302, 612, 614, 616, 618) das zweite Antriebselement (54, 60, 208, 308, 502, 504, 602, 604, 606, 608) rotiert.

6. Vorrichtung nach Anspruch 1, **dadurch gekennzeichnet, dass** das erste Antriebselement (62, 202, 302, 612, 614, 616, 618) mindestens einen beweglichen und/oder verformbaren Aktor (316a, 316b) umfasst, der so bewegbar und/oder verformbar ist, dass der Aktor (316a, 316b) eine Mantelfläche des zweiten Antriebselements (54, 60, 208, 308, 502, 504, 602, 604, 606, 608) zumindest zeitweise derart kontaktiert, dass durch Kraftschluss und/oder Formschluss zwischen dem Aktor (316a, 316b) und der Mantelfläche des zweiten Antriebselements (54, 60, 208, 308, 502, 504, 602, 604, 606, 608), die Kraft auf das zweite Antriebselement (54, 60, 208, 308, 502, 504, 602, 604, 606, 608) ausübbar ist.

7. Vorrichtung nach Anspruch 6, **dadurch gekennzeichnet, dass** der Aktor (316a, 316b) mindestens ein piezoelektrischer Aktor (316a, 316b) ist und mit dem, durch Kraftschluss und/oder Formschluss zwischen dem Aktor (316a, 316b) und der Mantelfläche des zweiten Antriebselements (54, 60, 208, 308, 502, 504, 602, 604, 606, 608), die Kraft auf das zweite Antriebselement (54, 60, 208, 308, 502, 504, 602, 604, 606, 608) ausübbar ist.

8. Vorrichtung nach einem der vorhergehenden Ansprüche, **dadurch gekennzeichnet, dass** die dem zweiten Antriebselement (54, 60, 208, 308, 502, 504, 602, 604, 606, 608) zugewandte Oberfläche des ersten Antriebselements (62, 202, 302, 612, 614, 616, 618) entlang eines Kreisbogens um die Rotationsachse (52) mit einem Mittelpunktswinkel von 45° bis 180° verläuft.

9. Vorrichtung nach einem der vorhergehenden Ansprüche, **dadurch gekennzeichnet, dass** die Sterilbarriere (42, 64) zumindest im Bereich zwischen dem ersten Antriebselement (62, 202, 302, 612, 614, 616, 618) und dem zweiten Antriebselement (54, 60, 208, 308, 502, 504, 602, 604, 606, 608) eine durchgehend geschlossene Oberfläche hat.

10. Vorrichtung nach einem der vorhergehenden Ansprüche, **dadurch gekennzeichnet, dass** ein Abschnitt der zwischen dem ersten Antriebselement (62, 202, 302, 612, 614, 616, 618) und dem zweiten Antriebselement (54, 60, 208, 308, 502, 504, 602, 604, 606, 608) angeordneten Sterilbarriere (42, 64) entlang eines Kreisbogens um die Rotationsachse (52) herum verläuft.

11. Vorrichtung nach einem der vorhergehenden Ansprüche, **dadurch gekennzeichnet, dass** die Koppeleinheit (44a, 204, 304, 610) mindestens eine Sensoreinheit zum Detektieren eines Drehwinkels des zweiten Antriebselements (54, 60, 208, 308, 502, 504, 602, 604, 606, 608) umfasst.

12. Vorrichtung nach Anspruch 11, **dadurch gekennzeichnet, dass** die Sensoreinheit einen optischen Sensor umfasst, der ein in einer Rotationsebene (55) umlaufendes, codiertes, optisch erfassbares Muster (212, 310) auf dem zweiten Antriebselement (54, 60, 208, 308, 502, 504, 602, 604, 606, 608) erfasst, wobei jedem erfassbaren Drehwinkel des zweiten Antriebselements (54, 60, 208, 308, 502, 504, 602, 604, 606, 608) ein bei diesem Drehwinkel durch den Sensor erfassbarer Teil des Musters (212, 310) zugeordnet ist, wobei sich jeder durch den Sensor bei einem Drehwinkel erfassbare Teil des Musters (212, 310) eindeutig von anderen durch den Sensor bei anderen Drehwinkeln erfassbaren Teilen des Musters (212, 310) unterscheidet.

13. Vorrichtung nach einem der vorhergehenden Ansprüche, **dadurch gekennzeichnet, dass** in einem gekoppelten Zustand die zweiten Antriebselemente (54, 60, 208, 308, 502, 504, 602, 604, 606, 608) in mehreren Rotationsebenen (55) übereinander entlang derselben Rotationsachse (52) angeordnet sind und jedes erste Antriebselement (62, 202, 302, 612, 614, 616, 618) in einer Rotationsebene (55) mit einem zweiten Antriebselement (54, 60, 208, 308, 502, 504, 602, 604, 606, 608) paarweise angeordnet ist, wobei die Anzahl der Paare von Antriebselementen mindestens zwei ist, insbesondere drei oder vier ist.

14. Vorrichtung nach Anspruch 13, **dadurch gekennzeichnet, dass** mehrere Sensoreinheiten vorgesehen sind, dass die Anzahl der Sensoreinheiten mindestens der Anzahl von zweiten Antriebselementen (54, 60, 208, 308, 502, 504, 602, 604, 606, 608) entspricht, und dass jeweils mindestens eine Sensoreinheit den Drehwinkel eines zweiten Antriebselements (54, 60, 208, 308, 502, 504, 602, 604, 606, 608) erfasst.

15. Vorrichtung nach einem der vorhergehenden Ansprüche, **dadurch gekennzeichnet, dass** die Instrumenteneinheit (100a bis 100d, 200, 300, 500, 600) ein Instrument mit einem an einem distalen Ende (57) eines Instrumentenschafts angeordneten Endeffektor (506, 610) umfasst, wobei das mindestens eine zweite Antriebselement (54, 60, 208, 308, 502, 504, 602, 604, 606, 608) mit dem Endeffektor (506, 610) gekoppelt ist und wobei der Endeffektor (506, 610) mit Hilfe des mindestens einen zweiten Antriebselements (54, 60, 208, 308, 502, 504, 602, 604, 606, 608) in mindestens einem Freiheitsgrad bewegbar und/oder steuerbar ist, insbesondere bei vier zweiten Antriebselementen (54, 60, 208, 308, 502, 504, 602, 604, 606, 608) in vier Freiheitsgraden bewegbar ist, wobei jeweils zwei zweite Antriebselemente (54, 60, 208, 308, 502, 504, 602, 604, 606, 608) eine Drehbewegung um die Längsachse des Instrumentenschafts bewirken und jeweils zwei weitere zweite Antriebselemente (54, 60, 208, 308, 502, 504, 602, 604, 606, 608) eine Längsbewegung in Richtung der Längsachse des Instrumentenschafts bewirken, oder
dass die Instrumenteneinheit (100a bis 100d, 200, 300, 500, 600) ein Endoskop mit einem Endoskopschaft umfasst, wobei das mindestens eine zweite Antriebselement (54, 60, 208, 308, 502, 504, 602, 604, 606, 608) mit dem Endoskop, dem Endoskopschaft und/oder einer Optik des Endoskops derart gekoppelt ist, dass mit Hilfe des mindestens einen zweiten Antriebselements (54, 60, 208, 308, 502, 504, 602, 604, 606, 608) in mindestens einem Freiheitsgrad eine Bewegung des Endoskops, des Endoskopschafts und/oder der Optik möglich ist.
